# EUROPÄISCHE PATENTSCHRIFT

(11) **EP 1 099 091 B1**
(45) Veröffentlichungstag und Bekanntmachung des Hinweises auf die Patenterteilung: **30.03.2005**
(21) Anmeldenummer: 99939408.3
(22) Anmeldetag: 27.07.1999
(51) Int. Cl.: G01B 3/16, A61C 19/04

(54) **MESSGERÄT IN ART EINES ZIRKELS, INSBESONDERE ZUR VERWENDUNG IN DER MEDIZINTECHNIK**
COMPASS-TYPE MEASURING DEVICE, NOTABLY FOR USE IN MEDICAL TECHNOLOGY
INSTRUMENT DE MESURE DE TYPE COMPAS, DESTINE NOTAMMENT A ETRE UTILISE DANS LE MATERIEL MEDICAL

(30) Priorität: 27.07.1998 DE 19833685
(43) Veröffentlichungstag der Anmeldung: 16.05.2001
(73) Patentinhaber: Akhavan-Sigari, Soheyl Dr., 81667 München (DE)
(72) Erfinder: AKHAVAN-SIGARI, Soheyl, D-37081 Göttingen (DE); HARUN-MAHDAVI, Sasan, D-80538 München (DE)
(86) Internationale Anmeldenummer: PCT/EP1999/005367
(87) Internationale Veröffentlichungsnummer: WO 2000/006968

(56) Entgegenhaltungen:
- DE-A- 3 502 556
- DE-A- 3 807 035
- DE-A- 4 422 721
- DE-C- 19 633 026
- GB-A- 2 278 316
- GB-A- 2 278 684

## Beschreibung

Die Erfindung betrifft ein elektronisches Rechengerät mit einer Displayeinrichtung und ein Meßgerät in Art eines Zirkels mit zwei um eine gemeinsame Drehachse verschwenkbaren Schenkeln, deren jeweilige Winkelstellung elektronisch abgreifbar und zur Längenmessung zwischen den Spitzen der Schenkel oder zur Messung des von den Schenkeln gebildeten Winkels mittels einer digitalen Anzeigeeinheit ablesbar ist, insbesondere zur Verwendung in der Medizintechnik.

In der Zahnmedizin sind manuelle Zirkel zur Abstandsmessung bekannt. Mittels eines zugehörigen Meßlineals besteht die Möglichkeit, Längen und Winkel zu bestimmen, um daraus relevante Daten bei der Auswertung von am Patienten, an Modellen oder mit Röntgenbildern gemessenen Größen zu gewinnen. Mit dieser bekannten Methode ist ein erheblicher Zeitaufwand verbunden; außerdem können beim Umgang mit derartigen Zirkeln und deren Auswertung mit dem Meßlineal erhebliche Ungenauigkeiten nicht ausgeschlossen werden.

Ein Meßgerät der eingangs genannten Art ist aus der deutschen Offenlegungsschrift 4422721 bekannt. Ein derartiger dentaler Winkelsteller erfaßt die Winkeländerung über elektronische Winkelgeber. Der Winkelwert wird digital angezeigt. Für die richtige Positionierung des Geräts ist eine umständliche Einspannung in einem Befestigungsadapter erforderlich. Die GB-A-2 278 316 zeigt einen elektronischen Zirkel mit Entfernungsanzeige. Figur 1b in der DE-A1-35 02 556 zeigt einen Zirkel mit einer Schenkel verbindung.

Demgegenüber liegt der vorliegenden Erfindung die Aufgabe zugrunde, ein universelles Meßgerät in Art eines Zirkels der eingangs genannten Art zu schaffen, welches sich insbesondere zur Verwendung in der Medizintechnik eignet; zu diesem Zweck ist es ungebunden, d.h. von Einspannvorrichtungen unabhängig anwendbar; es soll sich insbesondere durch eine einfache Handhabung und die rasche Auswertung der gewonnenen Meßgrößen auszeichnen. Diese Aufgabe wird nach den unabhängigen Ansprüchen 1, 6 gelöst.

Ein erfindungsgemäßes Meßgerät mit diesen Eigenschaften umfaßt zwei um eine gemeinsame Drehachse verschwenkbare Schenkel, deren jeweilige Winkelstellung zueinander elektronisch abgreifbar und zur Längenmessung zwischen den Spitzen der Schenkel oder zur Messung des von den Schenkeln gebildeten Winkels mittels einer digitalen Anzeigeeinheit ablesbar ist, insbesondere zur Verwendung in der Medizintechnik.

Dabei ist gemäß einer konkreten Ausführungsform die Drehachse durch ein Drehlager gebildet, welches zwei konzentrische Lagerschalen umfaßt; jede Lagerschale ist nur mit einem der beiden Schenkel verbunden und das Drehlager ist in einem Gehäuse aufgenommen, welches neben der Anzeigeeinheit eine elektronische Recheneinheit zur Auswertung der Meßdaten aufweist.

Die Recheneinheit ist so programmiert, daß für jede Schenkelposition der zwischen den Schenkeln eingeschlossene Winkel und wahlweise auch der Spitzenabstand unmittelbar auf der Anzeigeeinheit ablesbar ist.

Dadurch, daß die Recheneinheit in einer weiteren Ausgestaltung eine Speichereinheit zur Zwischenspeicherung und/oder Weiterverarbeitung der Meßdaten nach einem Anwenderprogramm umfaßt, ist sichergestellt, daß die gemessenen Daten gesichert und gemäß dem Anwenderprogramm, z. B. nach den vier Grundrechnungsarten, weiterverarbeitet werden können.

Das erfindungsgemäße Meßgerät kann auch wie ein üblicher elektronischer Rechner eingesetzt werden, wobei zum Eingeben von Rechengrößen zwei gegenseitig verschwenkbare, Winkelschwenkel bildende Stabelemente vorgesehen sind, deren Winkelstellung zueinander elektronisch abgreifbar ist, daß die hierbei erzeugten Signale Rechengrößen bilden, welche dem jeweils eingestellten Winkel entsprechen und daß weitere Rechengrößen aus Abständen zwischen den Stabelementen, jeweils bezogen auf einen eingestellten Winkel und definierten Abstandsendpunkten auf den Stabelementen, gebildet werden. Dabei kann die Stellenzahl der Rechengrößen unterschiedlich gewählt und die Komastelle, z. B. mit einer Taste, beliebig festgelegt werden.

Neben deren Funktion zur Betätigung des Rechners können die Stabelemente vorteilhaft als Lineale ausgebildet sein, die mit einer Spitze enden, welche in der Ebene der Auflagefläche liegt. Die Lineale sind bevorzugt aus transparentem Werkstoff gefertigt und besitzen eine Skalierung. Somit eignet sich der Rechner ganz besonders für eine Verwendung im Schulbetrieb als kombiniertes Rechen- und Zeicheninstrument.

In weiterer Ausgestaltung der Erfindung ist vorgesehen, daß die Recheneinheit mit einer Schnittstelle zu einer externen Datenverarbeitungsanlage versehen ist. Auf diese Weise können die Meßdaten auch in komplexeren Datenverarbeitungsprogrammen verwertet werden, und es können umfangreiche Protokolle erstellt werden.

Ein bevorzugter Ort zum elektronischen Abgreifen der Winkelstellung zwischen den beiden Schenkeln ist dadurch gegeben, daß die relative Drehlage der beiden Lagerschalen zueinander abgetastet wird. Dies kann mittels eines üblichen Potentiometers erfolgen; denkbar sind aber auch induktive, kapazitive, elektrooptische oder magnetische Winkelgeber.

Das Drehlager mit den beiden Lagerschalen befindet sich bevorzugt in einem Gehäuse, in welches die von den Spitzen entfernten Enden der Schenkel münden. Ein derartiges Gehäuse kann einteilig ausgebildet und mit einer der beiden Lagerschalen verbunden sein. Es kann auch als zweiteiliges Gehäuse ausgebildet sein, dessen Teile jeweils mit einer anderen Lagerschale verbunden sind. Dabei sind die Schenkel jeweils mit einer Lagerschale oder einem Gehäuseteil bevorzugt lösbar verbunden. Die lösbare Verbindung kann als Steckverbindung ausgebildet sein. Die Schenkelteile mit den Spitzen können auf diese Weise einfach entfernt und im Falle einer medizinischen Anwendung sterilisiert werden.

Durch eine Anordnung der Steckverbindung im Inneren des Gehäuses ist gewährleistet, daß Patienten nicht mit unsterilen Teilen des Meßgeräts in Berührung kommen. Das Gehäuse selbst kann zu diesem Zweck in einer Hülle aus einer transparenten sterilen Folie verpackt werden.

Zur Vereinfachung der Handhabung des Meßgeräts ist nach einer weiteren Ausgestaltung vorgesehen, daß die Schenkel durch eine im Gehäuse angeordnete Feder in einer gespreizten Grundstellung gehalten sind. Für Anwendungen in der Zahnmedizin kann diese Grundstellung z. B. bei einem Winkel von 120° erreicht sein. Für größere Spreizwinkel sind an den Schenkeln etwa senkrecht zur Drehachse nach außen vorspringende Fingermitnehmer vorgesehen. Diese sind bevorzugt gegen Spreizfedern an die Schenkel anklappbar oder in diese einklappbar ausgebildet.

Wird das Meßgerät nicht verwendet, so können die zusammengeklappten Schenkel mit ihren Spitzen voran in eine Kappe gesteckt werden, welche als Spitzenschutz dient und gleichzeitig Verletzungen vorbeugt.

Die mathematische Schenkellänge beträgt bevorzugt bis zu 20 cm; die maximale Schenkelöffnung beträgt bevorzugt 180°. Für Messungen am Patienten besitzen die Schenkel ein stumpfes Ende; bei Vermessungen von Röntgenbildern oder Messungen an Modellen verwendet man vorteilhafterweise Schenkel mit spitzen Enden.

Aus ergonometrischen Überlegungen ist es zweckmäßig, daß das Gehäuse als flacher Körper ausgebildet ist, dessen Umriß auf einem den Schenkelspitzen gegenüberliegenden Außenabschnitt passend für eine Griffposition zwischen Daumen und Zeigefinger geformt ist. Zu diesem Zweck kann der Außenabschnitt des Gehäuses etwa einen Kreisbogen beschreiben; ein derartiges Gehäuse läßt sich einfacher herstellen als ein oval geformtes Gehäuse, welches hingegen besser in die Hautfalte zwischen Daumen und Zeigefinger paßt.

Um den Halt in der Griffposition noch weiter zu verbessern, ist vorgesehen, daß die seitliche Umfangsfläche des Gehäuses wenigstens in dessen Außenbereich rinnenförmig nach innen geformt ist, so daß es die genannte Hautfalte einfaßt.

Damit das Gehäuse stets eine zentrale Position zwischen den Schenkeln beibehält kann eine die beiden Schenkel zentrierende Führungseinrichtung, wie man sie auch bei üblichen Zirkeln findet, vorgesehen sein.

Bei der Vermessung ebener Vorlagen wie beispielsweise von Röntgenbildern ist es zweckmäßig, daß das Gehäuse auf einer Seite mit einer Auflagefläche zur Durchführung von Messungen auf einer ebenen Unterlage versehen ist, welche mit den entsprechend abgebogenen Spitzen der Schenkel eine Ebene beschreibt.

Eine sichere Meßposition des Meßgeräts ist dadurch erzielbar, daß das Gehäuse ein durchgehendes Sichtfenster im Bereich um die Drehachse herum aufweist. Der Messende kann auf diese Weise durch das gesamte Gehäuse hindurchblicken und somit das Meßgerät auf einen bestimmten Scheitelpunkt des Objekts ausrichten. Das genannte Sichtfenster befindet sich bevorzugt innerhalb der genannten Auflagefläche. Ein Befestigungsclip kann entweder am Gehäuse auf der Seite der Auflagefläche oder an der Kappe, in welcher die beiden Schenkelspitzen aufgenommen sind, vorgesehen sein.

Für den praktischen Einsatz des erfindungsgemäßen Meßgeräts ist die Ausgestaltung der bevorzugt im Gehäuse untergebrachten Anzeige- und Bedieneinheit wesentlich. Dementsprechend ist im Gehäuse eine Bedieneinheit mit Betätigungstasten für die Ein/Ausschaltung, die Wahl der Maßeinheit und der Rechenoperation oder für den Datentransfer in den Speicher vorgesehen. Außerdem enthält das Gehäuse selbstverständlich ein Batteriefach für den Betrieb der Recheneinheit, welches mit einem Schraubdeckel verschlossen ist.

Wenn ein erster Meßwert durch eine "enter"-Taste gespeichert wird, so kann er anschließend mit einem weiteren Meßwert einer vorher durch Betätigen der "mode"-Taste eingegebenen Rechenoperation unterworfen werden usw. Zweckmäßig sind dabei die vier Grundrechenarten, die sowohl bei medizinischen Anwendungen erforderlich sind als auch bei Verwendung des erfindungsgemäßen Meßgeräts als Taschenrechner. Das Meßgerät befindet sich bevorzugt nach jedem Einschalten in einem Leerzustand, d.h. alle bis dahin gemessenen oder errechneten Werte werden durch das vorherige Ausschalten des Gerätes gelöscht.

Das erfindungsgemäße Meßgerät eignet sich nicht nur für Anwendungen in der Zahnmedizin; Schenkellängen, welche bis zu 20 cm betragen, ermöglichen dessen Einsatz auch in der Kieferorthopädie, z. B. zur Ermittlung des Gesichtslängenindex, in der Prothetik z. B. zur Ermittlung des Sprechabstandes und der Bißhöhe am Patienten, zur Vermessung von Muttermalen in der Dermatologie, zur Winkelmessung in der Orthopädie oder zur Vermessung von Referenzlängen und -abständen in der Gesichtschirurgie.

Im folgenden werden Ausführungsbeispiele der Erfindung anhand der Zeichnung erläutert. Es zeigt
- Fig. 1: eine schematische Draufsicht auf das erfindungsgemäße Meßgerät,
- Fig. 2: dessen Ansicht von unten,
- Fig. 3: dessen Seitenansicht,
- Fig. 4: eine weitere Ausführungsform in der Draufsicht,
- Fig. 5: die Ausführungsform gemäß Fig. 4 in der Seitenansicht,
- Fig. 6: eine Draufsicht auf eine Ausführungsform mit Linealen und
- Fig. 7: eine Seitenansicht zu Fig. 6 gemäß Pfeil VII.

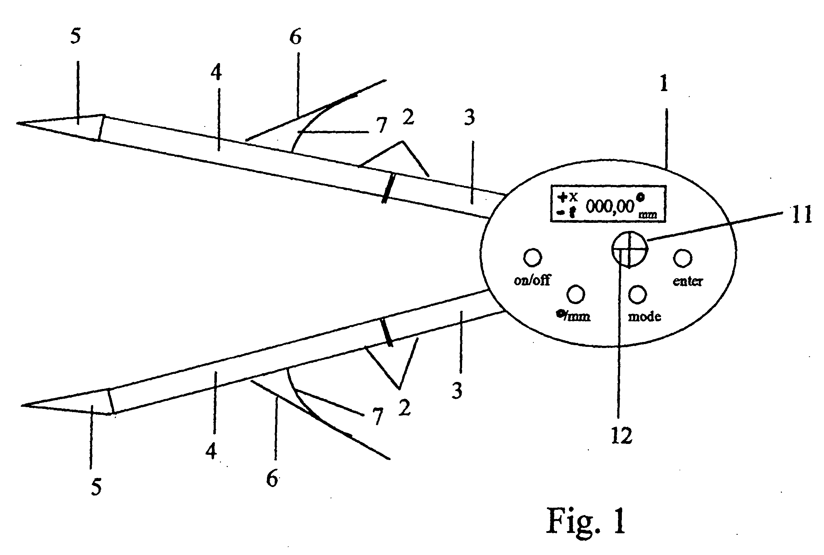

Nach der Ausführungsform des Meßgeräts gemäß den Fig. 1 bis 3 besitzt es im Scheitelpunkt der Schenkel 2 ein Gehäuse 1, auf dessen Oberseite Tasten zur Betätigung der darin untergebrachten Recheneinheit angeordnet sind.

Die "on/off"-Taste betrifft die Ein-/Ausschaltung des Rechners; mit der "°/mm"-Taste wird die auf dem Display angezeigte Maßeinheit ausgewählt; die "mode"-Taste ermöglicht das Auswählen einer der vier Grundrechenarten wie sie auf dem Display angezeigt sind, nämlich +, -, • und :. Die "enter"-Taste ermöglicht das Einspeichern des auf dem Display angezeigten Meßwerts, der insgesamt mit fünf Stellen angegeben werden kann.

In seinem Zentrum besitzt das Gehäuse 1 ein Sichtfenster 11 mit Fadenkreuz 12, damit die mathematische Achse der Schenkelverstellung genau in Übereinstimmung mit einer entsprechenden Markierung auf eine Unterlage gebracht werden kann. Das Sichtfenster 11 ist nicht als bloße Öffnung oder Durchbrechung ausgebildet, sondern beidseitig durch transparente Abdeckungen verschlossen, um ein Verschmutzen zu vermeiden.

zwei Schenkelstummel 3 sind im Inneren des Gehäuses an gegeneinander verdrehbaren, nicht gezeigten Lagerschalen befestigt. An ihren freien Enden sitzen lösbare Schenkelteile 4 mit Spitzen 5. Im Bereich der lösbaren Schenkelteile 4 sind Fingermitnehmer 6 vorgesehen, die mittels Spreizfedern 7 ausgestellt sind. Bei Nichtgebrauch können die Fingermitnehmer 6 an die Schenkelteile 4 angedrückt werden, wo sie mittels einer nicht gezeigten Arretierung in dieser Position verbleiben.

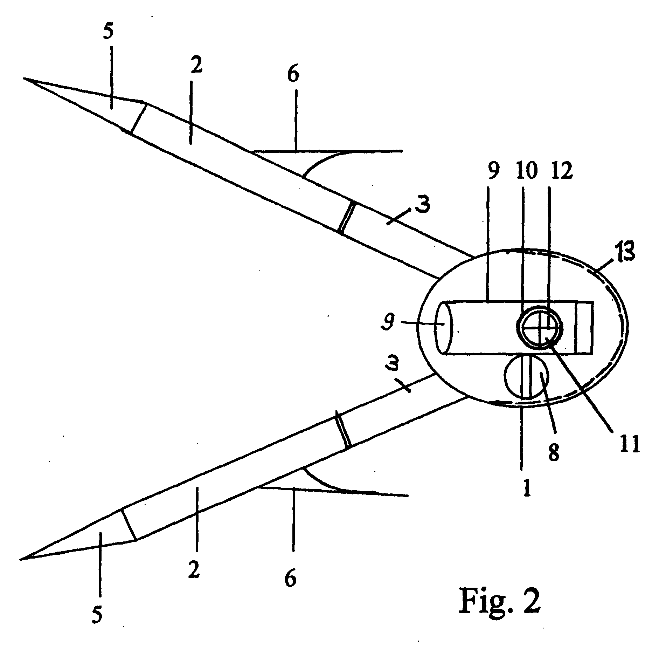

Auf der Ansicht des Gehäuses 1 von unten gemäß Fig. 2 erkennt man einen Schraubdeckel 8 für ein Batteriefach sowie ein federndes Clipteil 9 mit einem Ausschnitt 10 für das Sichtfenster 11, welches benachbart der Unterseite ein Fadenkreuz 12 aufweist.

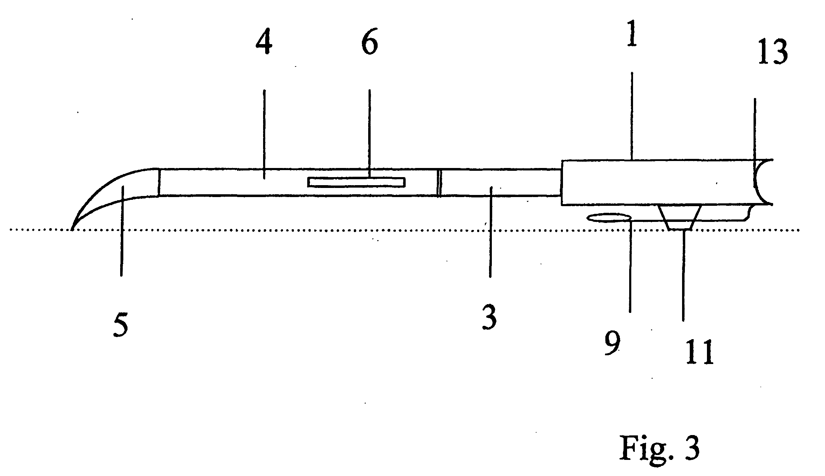

In der Seitenansicht gemäß Fig. 3 erkennt man die konkave Ausbildung des Außenabschnitts des Gehäuses 1, welches hier als einteiliges Gehäuse ausgebildet ist. In die dadurch gebildete Umfangsrinne 13 tritt beim Gebrauch des Meßgeräts die zwischen Zeigefinger und Daumen gebildete Hautfalte ein, so daß das Meßgerät dort eine sichere Anlage findet.

Gemäß Fig. 3 sind die Spitzen scharf ausgebildet und in Richtung auf die Rückseite gekrümmt, so daß die Enden der Spitzen in einer Ebene mit der Auflagefläche des Sichtfensters 11 liegen. Derartig ausgebildete Schenkel eignen sich besonders gut für Vermessungsarbeiten auf einer ebenen Unterlage, z. B. beim Vermessen von Röntgenbildern. Für Vermessungsarbeiten aus größerer Entfernung können die Spitzen durch Laserpointer ersetzt sein. Außerdem können ein oder beide Schenkel mit einer Wasserwaage versehen sein.

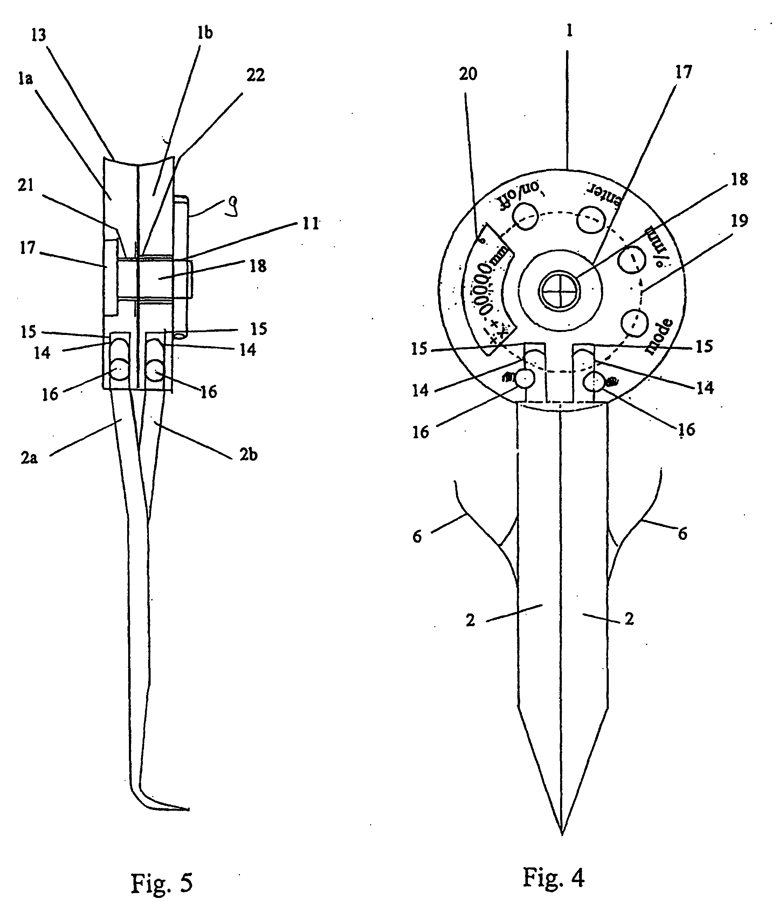

Bei der Ausführungsform gemäß den Fig. 4 und 5 ist das Gehäuse 1 horizontal geteilt in einen oberen Gehäuseteil 1a und einen unteren Gehäuseteil 1b. Ein Schenkel 2 ist mit einem nach oben geformten Anschlußende 2a in dem oberen Gehäuseteil 1a, der andere Schenkel 2 mit einem nach unten geformten Anschlußende 2b im unteren Gehäuseteil 1b befestigt. Zu diesem Zweck besitzen die Anschlußenden 2a, 2b rechteckige Paßstifte 14, mit denen sie in die entsprechenden Gehäusehülsen 15 eintreten, wo sie mittels federbelasteter Kugelrasten 16 gehalten sind. Die beiden Schenkel besitzen jeweils eine nach unten gebogene stumpfe Spitze, welche für das Abtasten am Patienten geeignet ist. Ein durchgehendes Sichtfenster 11 ist bei dieser Ausführungsform ebenfalls vorgesehen. Es ist aus einem Scheibenteil 17 und einem Röhrenteil 18 zusammengesetzt. Der Röhrenteil 18 sitzt innerhalb einer Lagerschale 21, die mit dem oberen Gehäuseteil 1a verbunden ist. Eine äußere Lagerschale 22 umgibt konzentrisch die innere Lagerschale 21. Die äußere Lagerschale 22 ist mit dem unteren Gehäuseteil 1b verbunden.

Die verschiedenen Betätigungstasten sind hier im oberen Gehäuseteil 1a längs einer Kreislinie 19 angeordnet; dies gilt ebenfalls für das Display, dessen Fenster 20 einen Kreisbogen beschreibt.

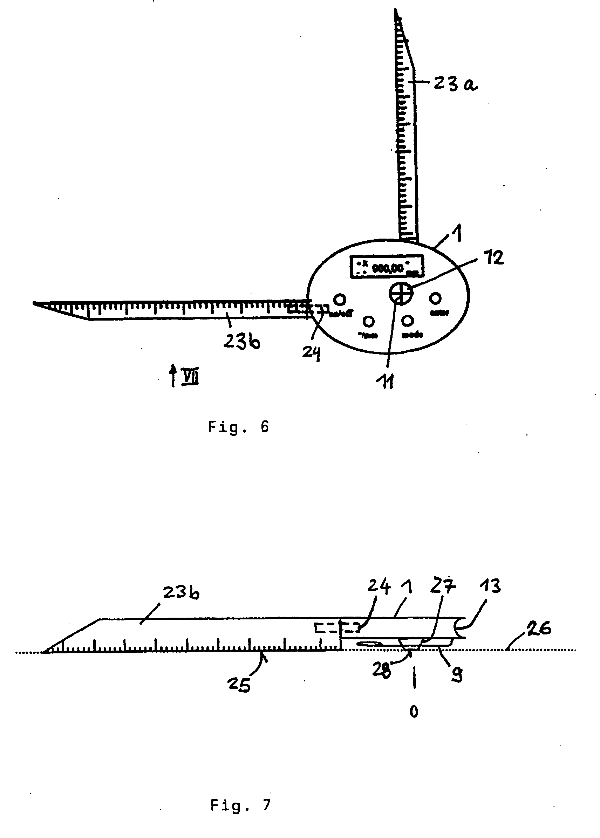

Die Fig. 6 und 7 zeigen eine Ausgestaltung als Rechen- und Zeichengerät, insbesondere für Schulzwecke. Die beiden Schenkel werden durch aufsteckbare Lineale 23a, 23b gebildet, deren Steckverbindungen durch Zapfen 24 verdeutlicht sind. In der Seitenansicht gemäß Fig. 7 ist dargestellt, daß die Lineale 23a, 23b ebene Auflageseiten 25 besitzen und jeweils mit einer Spitze in der Ebene der Auflageseiten 25 enden.

Gemäß Fig. 7 liegen die Lineale 23a, 23b somit satt auf einer Auflagefläche 26 auf. Dabei kann der Scheitelpunkt "O" des zwischen den Linealen 23a, 23b gebildeten Winkels einfach und genau mittels des Fadenkreuzes 11 im Sichtfenster 12 eingestellt werden. Hierzu ist hilfreich, daß das Sichtfenster 12 in einem Gehäusevorsprung 27 angeordnet ist, dessen Stirnfläche 28 ebenfalls auf der Auflagefläche 26 aufliegt.

## Patentansprüche

1. Elektronisches Rechengerät mit einer Displayeinrichtung und mehreren Rechenfunktionen,
bei dem zum Eingeben von Rechengrößen zwei gegenseitig verschwenkbare, Winkelschenkel bildende Stabelemente vorgesehen sind, wobei die Drehachse durch ein Drehlager gebildet ist, welches zwei konzentrische Lagerschalen umfasst, und deren Winkelstellung zueinander elektronisch abgreifbar ist, derart, daß die hierbei erzeugten Signale Rechengrößen bilden, welche dem jeweils eingestellten Winkel entsprechen,
bei dem weitere Rechengrößen aus Abständen zwischen den Stabelementen, jeweils bezogen auf einen eingestellten Winkel und definierten Abstandsendpunkten auf den Stabelementen gebildet werden, einstellbar sind und
welches Betätigungstasten zum Einspeichern des auf dem Display angezeigten Meßwerts (enter), zum Wählen von Grundrechenarten (mode) und der auf dem Display angezeigten Meßeinheit (°/mm) sowie einen Hauptschalter (on/off) aufweist.

2. Rechengerät nach Anspruch 1,
**dadurch gekennzeichnet,**
**daß** die Stabelemente als Lineale (23a, 23b) ausgebildet sind, die mit einer Spitze enden, welche in der Ebene ihrer Auflageseiten (25) liegt.

3. Rechengerät nach Anspruch 2,
**dadurch gekennzeichnet,**
**daß** die Lineale (23a, 23b) jeweils an ihrer unteren, an die Auflageseiten (25) angrenzenden Kante der dem anderen Lineal zugewandten Innenseite eine Skalierung aufweisen.

4. Rechengerät nach Anspruch 2,
**dadurch gekennzeichnet,**
**daß** die Innen- und Auflageseiten (25) der Lineale (23a, 23b) bis zur Spitze eben ausgebildet sind.

5. Rechengerät nach Anspruch 1,
**dadurch gekennzeichnet,**
**daß** es im Bereich der geometrischen Schwenkachse der Stabelemente durchschaubar ausgebildet und mit einer Achsenmarkierung versehen ist.

6. Meßgerät in Art eines Zirkels mit zwei um eine gemeinsame Drehachse verschwenkbaren Schenkeln (2), deren jeweilige Winkelstellung zueinander elektronisch abgreifbar und zur Längenmessung zwischen den Spitzen (5) der Schenkel (2) oder zur Messung des von den Schenkeln (2) gebildeten Winkels mittels einer digitalen Anzeigeeinheit ablesbar ist, insbesondere zur Verwendung in der Medizintechnik,
**dadurch gekennzeichnet,**
**daß** die Drehachse durch ein Drehlager gebildet ist, welches zwei konzentrische Lagerschalen umfaßt,
**daß** jede Lagerschale direkt oder indirekt nur mit einem der beiden Schenkel (2) verbunden ist und
**daß** das Drehlager in einem Gehäuse (1) aufgenommen ist, welches neben der Anzeigeeinheit eine elektronische Recheneinheit zur Auswertung der Meßdaten aufweist.

7. Meßgerät nach Anspruch 6,
**dadurch gekennzeichnet,**
**daß** die Recheneinheit eine Speichereinheit zur Zwischenspeicherung und/oder Weiterverarbeitung der Meßdaten nach einem Anwenderprogramm umfaßt.

8. Meßgerät nach Anspruch 6,
**dadurch gekennzeichnet,**
**daß** die Recheneinheit mit einer Schnittstelle zu einer externen Datenverarbeitungsanlage versehen ist.

9. Meßgerät nach Anspruch 6,
**dadurch gekennzeichnet,**
**daß** das Gehäuse (1) einteilig ausgebildet und mit einer der beiden Lagerschalen verbunden ist.

10. Meßgerät nach Anspruch 6,
**dadurch gekennzeichnet,**
**daß** das Gehäuse (1) zweiteilig ausgebildet ist, dessen Teile (1a, 1b) jeweils mit einer Lagerschale (21, 22) verbunden sind.

11. Meßgerät nach Anspruch 9 oder 10,
**dadurch gekennzeichnet,**
**daß** die Schenkel (2) jeweils entweder direkt mit einer Lagerschale oder einem Gehäuseteil (1a, 1b) lösbar verbunden sind.

12. Meßgerät nach Anspruch 11,
**dadurch gekennzeichnet;**
**daß** die lösbare Verbindung als Steckverbindung ausgebildet ist.

13. Meßgerät nach Anspruch 6,
**dadurch gekennzeichnet,**
**daß** die Schenkel (2) durch eine im Gehäuse (1) angeordnete Feder in einer gespreizten Grundstellung gehalten sind.

14. Meßgerät nach Anspruch 6,
**dadurch gekennzeichnet,**
**daß** die Schenkel (2) etwa senkrecht zur Drehachse nach außen vorspringende Fingermitnehmer (6) aufweisen.

15. Meßgerät nach Anspruch 14,
**dadurch gekennzeichnet,**
**daß** die Fingermitnehmer (6) gegen Spreizfedern (7) an die Schenkel anklappbar oder in diese einklappbar sind.

16. Meßgerät nach Anspruch 14,
**dadurch gekennzeichnet,**
**daß** die Fingermitnehmer (6) in Schenkellängsrichtung verstellbar angeordnet sind.

17. Meßgerät nach Anspruch 6,
**dadurch gekennzeichnet,**
**daß** im Gehäuse (1) eine Bedieneinheit mit Betätigungstasten für die Ein-/Ausstellung, die Wahl der Recheneinheit und der Rechenoperation, oder für den Datentransfer in den Speicher vorgesehen ist.

18. Meßgerät nach Anspruch 6,
**dadurch gekennzeichnet,**
**daß** das Gehäuse (1) als flacher Körper ausgebildet ist, dessen Umriß auf einem den Schenkelspitzen gegenüberliegenden Außenabschnitt passend für eine Griffposition zwischen Daumen und Zeigefinger geformt ist.

19. Meßgerät nach Anspruch 18,
**dadurch gekennzeichnet,**
**daß** der Außenabschnitt des Gehäuses (1) etwa einen Kreisbogen beschreibt.

20. Meßgerät nach Anspruch 18,
**dadurch gekennzeichnet,**
**daß** der Außenabschnitt des Gehäuses (1) bei einem einteiligen Gehäuse oval geformt ist.

21. Meßgerät nach Anspruch 18,
**dadurch gekennzeichnet,**
**daß** die seitliche Umfangsfläche des Gehäuses (1) wenigstens in dessen Außenbereich rinnenförmig nach innen geformt ist.

22. Meßgerät nach Anspruch 18,
**dadurch gekennzeichnet,**
**daß** das Gehäuse (1) auf einer Seite mit einer Auflagefläche zur Durchführung von Messungen auf einer ebenen Unterlage versehen ist, welche mit den Spitzen (5) der Schenkel (2) eine Ebene beschreibt.

23. Meßgerät nach Anspruch 18,
**dadurch gekennzeichnet,**
**daß** das Gehäuse (1) ein durchgehendes Sichtfenster (11) im Bereich um die Drehachse herum aufweist.

## Claims

1. Electronic computer with a display feature and several computing functions on which two reciprocally swinging rod elements forming an angle leg are provided for inputting operands, wherein the axis of rotation is formed from a pivot bearing comprising two concentric bearing seats with angles that can be electronically measured together such that the signals thus generated forms operands, which correspond to the respectively set angle, on which further operands formed from distances between the rod elements, in each case relative to a set angle and defined distance points on the rod elements and which exhibit operating keys for saving the readout value indicated in the display (enter), for selecting the basic computer types (mode) and the measuring unit indicated in the display (° / mm) and also a main switch (on/off).

2. Computer in accordance with Claim 1, **characterised in that** the rod elements are formed as rules (23a, 23b), which end in a point that lies at the level of its bearing sides (25).

3. Computer in accordance with Claim 2, **characterised in that** each of the rules (23a, 23b) exhibit a scale on the lower edge of the inside facing the opposite rule and adjacent to the bearing sides (25).

4. Computer in accordance with Claim 2, **characterised in that** the inside and bearing sides (25) of the rules (23a, 23b) are also formed to a point.

5. Computer in accordance with Claim 1, **characterised in that** in the area surrounding the geometric swivel axis, the rod element is transparent and exhibits an axis marking.

6. Measuring device of a circular type with two limbs rotatable about a common axis of rotation in (2), the angles of which can be electronically measured and read-off for measuring the length between the tips (5) of the limbs (2) or for measuring the angle formed from the limbs (2) using a digital display unit, specifically for use in medical technology, **characterised in that** the axis of rotation is formed by a pivot bearing comprising two concentric bearing seats, **in that** each bearing seat is connected, either directly or indirectly with only one of the two limbs (2) and **in that** the pivot bearing is encased in a housing (1), which, in addition to the display unit, also exhibits an computing unit for evaluating the read-out data.

7. Measuring device in accordance with Claim 6, **characterised in that** the computing unit comprises a storage unit for the clipboard storage and/or further processing of readout data according to a user program.

8. Measuring device in accordance with Claim 6, **characterised in that** the computing unit features an interface to an external data processing unit.

9. Measuring device in accordance with Claim 6, **characterised in that** the housing (1) is formed as a single-part component and is connected to one of the two bearing seats.

10. Measuring device in accordance with Claim 6, **characterised in that** the housing (1) is formed as a two-part component, each of its parts (1a, 1b) being connected to a bearing seat (21, 22).

11. Measuring device in accordance with Claim 9 or 10, **characterised in that** each limb (2) is connected either directly or indirectly to and can be detached from a bearing seat or a housing part (1a, 1b).

12. Measuring device in accordance with Claim 11, **characterised in that** the detachable connection is formed as a plug-and-socket connection.

13. Measuring device in accordance with Claim 6, **characterised in that** the limbs (2) are held in a splayed basic position by a spring arranged in the housing (1).

14. Measuring device in accordance with Claim 6, **characterised in that** the limbs (2) exhibit finger carriers positioned approx. vertical to the pivot axis and projecting outwards.

15. Measuring device in accordance with Claim 14, **characterised in that** the finger carriers (6) can be folded against or into the limb against bracing springs (7).

16. Measuring device in accordance with Claim 14, **characterised in that** the finger carriers (6) have an adjustable arrangement along the length of the limbs.

17. Measuring device in accordance with Claim 6, **characterised in that** the housing (1) accommodates an operating unit with operating keys for selecting/deselecting the computing unit and computing operation or for transferring data to the memory.

18. Measuring device in accordance with Claim 6, **characterised in that** the housing (1) is formed as a flat element, the contour of which is shaped on an outside section facing the limb tip, suitable for being held between thumb and index finger.

19. Measuring device in accordance with Claim 18, **characterised in that** the housing (1) is approximately circular.

20. Measuring device in accordance with Claim 18, **characterised in that** the outside section of a single-component housing (1) is oval.

21. Measuring device in accordance with Claim 18, **characterised in that** the lateral peripheral surface of the housing (1), at least in its outside area, is chamfered inwards.

22. Measuring device in accordance with Claim 18, **characterised in that** the housing (1) exhibits on one side a bearing surface for performing measurements on an even surface, which, together with the tips (5) of the limbs (2) depicts one level.

23. Measuring device in accordance with Claim 18, **characterised in that** the housing (1) exhibits a continuous viewing window (11) around the area of the rotating axis.

## Revendications

1. Machine à calculer électronique munie d'un dispositif d'affichage et de plusieurs fonctions de calcul pour laquelle sont prévues deux barres mutuellement orientables formant des branches angulaires pour la rentrée de paramètres, l'axe de rotation étant formé par un coussinet de pivotement comprenant deux coquilles de coussinets concentriques et dont la position angulaire l'une par rapport à l'autre est électroniquement ajustable, de sorte que les signaux ainsi produits créent des paramètres correspondant à l'angle respectivement réglé auquel d'autres paramètres se forment sur la barre à partir d'intervalles entre les barres, respectivement en rapport avec un angle réglé et des points finaux des intervalles définis, sont réglables et qui présente des touches de commande pour enregistrer la valeur indiquée sur le panneau d'affichage (enter), pour sélectionner les opérations élémentaires (mode) et l'unité de mesure indiquée sur le panneau d'affichage (°/mm) ainsi qu'un commutateur principal (on/off).

2. Machine à calculer selon la revendication 1,
**caractérisée en ce que**
les barres se présentent sous la forme de règles (23a, 23b) se terminant par une pointe placée sur le plan de leurs faces de support (25).

3. Machine à calculer selon la revendication 2,
**caractérisée en ce que**
les règles (23a, 23b) présentent un cadrage respectivement sur leur bord inférieur adjacent aux faces de support du côté intérieur tourné vers l'autre règle.

4. Machine à calculer selon la revendication 2,
**caractérisée en ce que**
les côtés intérieurs et de support (25) des règles (23a, 23b) sont plats jusqu'à la pointe.

5. Machine à calculer selon la revendication 1,
**caractérisée en ce que**
la zone de l'axe de pivotement géométrique des barres est visible et dotée d'un marquage.

6. Instrument de mesure du type compas muni de deux branches orientables sur un axe de rotation commun dans (2), dont la position angulaire respective l'une par rapport à l'autre est électroniquement ajustable et lisible pour la mesure des longueurs entre les pointes (5) des branches (2) ou pour la mesure de l'angle formé par les branches (2) au moyen d'un indicateur numérique, notamment prévu pour être utilisé dans le domaine de la technique médicale,
**caractérisé en ce que**
l'axe de rotation est formé par un coussinet de pivotement comprenant deux coquilles de coussinets concentriques,
que chaque coquille de coussinet est reliée directement ou indirectement avec une des deux branches (2) seulement et que le coussinet de pivotement est logé dans un boîtier (1) présentant, en plus de l'unité d'affichage, une unité de calcul électronique pour l'exploitation des données de mesure.

7. Instrument de mesure selon la revendication 6,
**caractérisé en ce que**
l'unité de calcul comprend une unité de mémorisation pour le stockage intermédiaire et/ou le traitement ultérieur des données de mesure d'après un programme d'application.

8. Instrument de mesure selon la revendication 6,
**caractérisé en ce que**
l'unité de calcul est dotée d'une interface vers un ordinateur externe.

9. Instrument de mesure selon la revendication 6,
**caractérisé en ce que**
le boîtier (1) est constitué en une seule pièce et est relié avec l'une des deux coquilles de coussinets.

10. Instrument de mesure selon la revendication 6,
**caractérisé en ce que**
le boîtier (1) est constitué de deux pièces dont les parties (1a, 1b) sont respectivement reliées avec une coquille de coussinet (21, 22).

11. Instrument de mesure selon l'une des revendications 9 ou 10,
**caractérisé en ce que**
les branches (2) sont chacune directement reliées soit avec une coquille de coussinet soit avec une partie du boîtier (1a, 1b) de manière à être détachables.

12. Instrument de mesure selon la revendication 11,
**caractérisé en ce que**
la liaison détachable se présente sous la forme d'une fiche de raccordement.

13. Instrument de mesure selon la revendication 6,
**caractérisé en ce que**
les branches (2) sont maintenues en position initiale écartée par un ressort disposé dans le boîtier (1).

14. Instrument de mesure selon la revendication 6,
**caractérisé en ce que**
les branches (2) présentent, en position à peu près verticale par rapport à l'axe de rotation, des doigts d'entraînement (6) faisant saillie vers l'extérieur.

15. Instrument de mesure selon la revendication 14,
**caractérisé en ce que**
les doigts d'entraînement (6) contre des ressorts écarteurs (7) sont repliables sur les branches ou escamotables dans celles-ci.

16. Instrument de mesure selon la revendication 14,
**caractérisé en ce que**
les doigts d'entraînement (6) sont disposés dans le sens de la longueur des branches, de manière à être réglés.

17. Instrument de mesure selon la revendication 6,
**caractérisé en ce que**
une unité de commande avec des touches est prévue dans le boîtier (1) pour l'arrêt/la mise en marche, le choix de l'unité de calcul et l'opération de calcul ou pour le transfert des données dans l'unité de stockage

18. Instrument de mesure selon la revendication 6,
**caractérisé en ce que**
le boîtier (1) se présente sous la forme d'un corps plat dont le côté de la partie extérieure opposée aux pointes des branches est adapté pour former une poignée entre le pouce et l'index.

19. Instrument de mesure selon la revendication 18,
**caractérisé en ce que**
la partie extérieure du boîtier (1) décrit approximativement un arc de cercle.

20. Instrument de mesure selon la revendication 18,
**caractérisé en ce que** la partie extérieure du boîtier (1) a une forme ovale quand il s'agit d'un boîtier en une pièce.

21. Instrument de mesure selon la revendication 18,
**caractérisé en ce que**
le côté latéral du boîtier (1) a une la forme d'une gouttière orientée vers l'intérieur, au moins dans sa zone extérieure.

22. Instrument de mesure selon la revendication 18,
**caractérisé en ce que**
le boîtier (1) est doté sur un côté d'une face de support pour réaliser des mesures sur un support plat, cette face décrivant un plan avec les pointes (5) des branches (2).

23. Instrument de mesure selon la revendication 18,
**caractérisé en ce que**
le boîtier (1) présente une ouverture (11) d'un seul tenant dans la zone située autour de l'axe de rotation.
